# EUROPEAN PATENT SPECIFICATION

(11) **EP 2 020 212 B1**
(45) Date of publication and mention of the grant of the patent: **31.08.2016**
(21) Application number: 08013604.7
(22) Date of filing: 29.07.2008
(51) Int. Cl.: A61B 18/14, A61B 90/00

(54) **Polyp removal jaws**
Polypenentfernungsklemme
Mâchoires à suppression de polype

(30) Priority: 30.07.2007 US 881946
(43) Date of publication of application: 04.02.2009
(60) Divisional application: 11179004.4
(73) Proprietor: Covidien LP, Mansfield, MA 02048 (US)
(72) Inventor: Artale, Ryan, Boulder CO 80305 (US); Carlton, John, Las Vegas NV 89118 (US)
(74) Representative: Soames, Candida Jane

(56) References cited:
- EP-A- 0 543 122
- EP-A- 0 795 301
- DE-A1- 4 130 064
- DE-U1- 9 215 589
- US-A- 4 671 274
- US-B1- 6 273 887

## Description

### BACKGROUND

### Technical Field

The present disclosure relates to an apparatus for the removal of internal tissue, and more particularly, to jaw members configured for the removal of polyps and the like. The technical features of the pre-characterizing past of claim 1 below are to be found in EP0795301.

### Background of Related Art

A polyp is an abnormal growth of tissue projecting from a mucous membrane. A polyp that is attached to the surface of the mucous membrane by a narrow elongated stalk is said to be pedunculated. If no stalk is present, the polyp is said to be sessile. Polyps are commonly found in the colon, stomach, nose, urinary bladder and uterus. Polyps may also occur elsewhere in the body where mucous membranes exist like the cervix and small intestine.

The surgical procedure for removing a polyp is generally referred to as a "polypectomy". Polypectomys are generally endoscopic or laparoscopic procedures performed through the oral or anal cavities. When the location of the polyp permits, the polypectomy may be performed as an open procedure. Conventional polypectomys are completed using various apparatus and techniques known in the art.

As noted above, there are two forms of polyps, sessile and pedunculated. The stalkless or sessile polyps are generally removed using electrical forceps. The excess tissue projecting from the mucous membrane is cauterized, sealed, or the like, and torn from the tissue wall. Large sessile polyps or pedunculated polyps (e.g., polyps having stalks) tend to be relatively larger with a greater blood supply. The size and shape of large sessile polyps or pedunculated polyps typically do not lend themselves to being removed using traditional forceps. Unlike sessile polyps, large sessile polys or pedunculated polyps cannot simply be grasped in the jaw members of an electric forceps and torn from the tissue wall. Instead, the polypectomy is performed using a surgical snare device. The snare device is configured with a snare for looping over the hanging polyp and fitting securely over the polyp and/or poly stalk. By constricting the snare, and selectively applying energy, the device may cauterize or seal the polyp along the stalk as the polyp is severed from the tissue wall.

Polyp removal using a surgical snare device requires an operator to loop the snare over the end of the polyp in order to properly position the polyp about the stalk. In many circumstances access to the stalk of the polyp, much less the entire polyp is limited. Without complete access to the polyp the surgical snare device is useless for removal of the polyp.

It would therefore be beneficial to have a polyp removal device that does not incorporate a snare that must be placed looped over a polyp.

Attention is invited to the following documents as disclosures of electro-surgical devices: US-A-4671274, DE-A1-4130064, DE-U1-9215589.8, US-A-6273887, EP-A-795301 and 543122.

### SUMMARY

The present disclosure relates tp apparatus and methods for the removal of polyps and the like. The invention is defined in claim 1 below and the dependent claims are directed to optional features and preferred embodiments.

The present disclosure is of an apparatus for the removal of tissue comprises a first jaw member including first and second elongated members, the first and second elongated members having proximal and distal ends and defining a channel therebetween; and a second jaw member having proximal and distal ends and defining a tissue contacting surface, the proximal end of the second jaw member being pivotably coupled between the proximal ends of the first and second elongated members, wherein the distal end of the second jaw member is in a spaced apart relationship with the first jaw member when in a first position, and the distal end of the second jaw member extends into the channel defined between the distal ends of first and second elongated members when in a second position.

The distal end of the second jaw member extends beyond the distal end of first and second elongated members of the first jaw member when the jaws are in the second position. The second jaw member can deliver electrosurgical energy to the tissue.

The first and second elongated members may include a shelf extending at least partially therebetween. An electrode may be supported on the shelf. The shelf may be configured to extend distally between the first and second elongated members to form a stop for engaging a distal end of the second jaw member when the jaws are in the second position. An electrode may be operably disposed between the first and second elongated members.

The first and second jaw members may be resilient. At least one of the first jaw member and the second jaw member may include a semi-arcuate shape. Proximal ends of the first and second jaw members may be configured to be operably engaged with an endoscopic device.

The first and second jaw members may be configured for bipolar sealing of tissue. The first jaw may be at least partially electrically conductive, and wherein the second jaw may be electrically non-conductive and includes at least one electrode disposed between the first and second elongated members thereof.

The second jaw member can be configured to operably engage the shelf when the first and second jaw members are in a closed position.

A gap may be formed between the first and second jaw members when the first and second jaw members are in a closed position. The first and second jaw members may be configured for a bipolar electrosurgical procedure. The second jaw member is configured for a monopolar electrosurgical procedure.

The first and second jaw members may be formed from a resilient material.

The apparatus may further include a housing for receiving the first and second jaw members therein. The first and second jaw members may be flexed for receipt within the housing.

A system for the removal of tissue is provided. The system comprises an pparatus as mentioned above, together with a source of electrosurgical energy operably connected to at least one of the jaw members to deliver electrosurgical energy to the tissue.

### BRIEF DESCRIPTION OF THE DRAWINGS

The foregoing summary, as well as the following detailed description will be better understood when read in conjunction with the appended figures. For the purpose of illustrating the present disclosure, a preferred embodiment is shown. It is understood, that the present disclosure is not limited to the precise arrangement and instrumentalities shown.
FIG. 1 is a perspective view of polyp removal jaws that lack the monopolar pencil of the claimed invention, in a first or open position;
FIG. 2 is a perspective view of the polyp removal jaws of FIG. 1, shown in a second or closed position;
FIG. 3 is partial, cross-sectional, side view of the polyp removal jaws of FIGS. 1 and 2, shown in a closed position, and shown operably connected to an endoscopic device;
FIG. 4 is a partial, cross-sectional, side view of the polyp removal jaws of FIGS. 1-3, shown in an open position, operably connected to an endoscopic device;
FIG. 5A is a cross-sectional, side view of the polyp removal jaws of FIGS. 1-4, shown in a closed position;
FIGS. 5B-5F are transverse, cross-sectional views of the polyp removal jaw of FIGS. 1-5A taken along lines 5B-5B, 5C-C, 5D-5D, 5E-5E and 5F-5F, respectively, of FIG. 5A;
FIG. 6 is a perspective view of other polyp removal jaws that are in accordance with the presently claimed invention, shown in a first or open position;
FIG. 7 is a perspective view of the polyp removal jaws of FIG. 6, shown in a second or closed position:
FIG. 8 is a perspective view of other polyp removal jaws that lack the monopolar pencil of the claimed invention, shown in first or open position;
FIG. 9 is a perspective view of the polyp removal jaws of FIG. 8, shown in a second or closed position;
FIG. 10A is a cross-sectional, side view of the polyp removal jaws of FIGS. 8-9, shown in a closed position;
FIGS. 10B-10E are transverse, cross-sectional views of the polyp removal jaws of FIGS 8-10A taken along line 10B-10B, 10C-10C, 10D-10D and 10E-10E, respectively, of FIG. 10A;
FIGS. 11A-11D are partial, cross-sectional, side views of polyp removal jaws shown at various stages of insertion into a tubular housing; and
FIG. 12 is a schematic illustration of an electrosurgical system including any of the jaws shown in FIGS. 1-11D.

### DETAILED DESCRIPTION OF EMBODIMENT

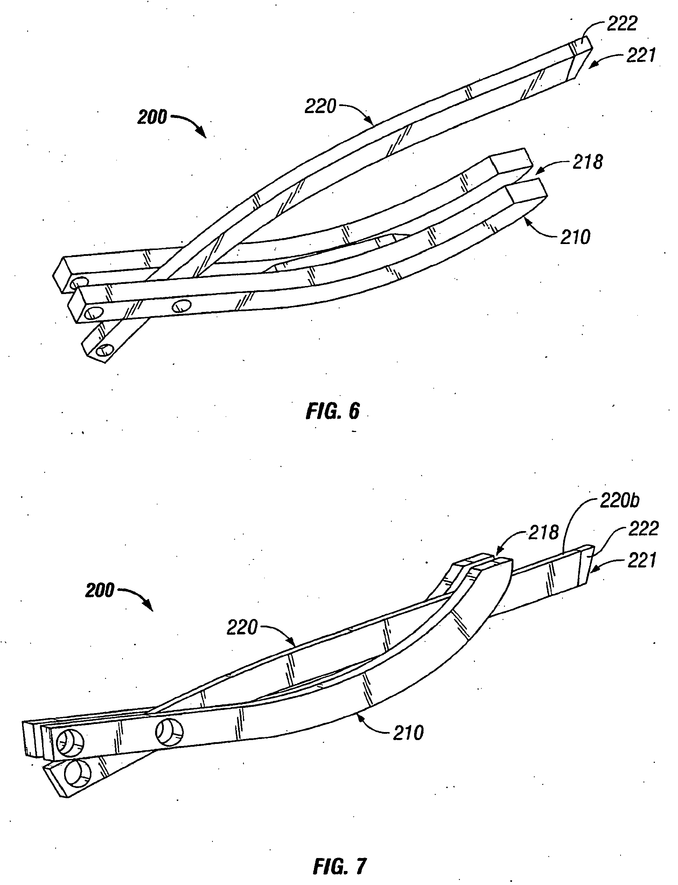

The foregoing summary, as well as the following detailed description will be better understood when read in conjunction with the appended figures. For the purpose of illustrating the present invention, attention is directed to drawing figures 6 and 7. It is understood, however, that the present disclosure is not limited to the precise arrangement and instrumentalities shown. As shown in the drawings and described throughout the following description, as is traditional when referring to relative positioning on an object, the term "proximal" refers to the end of the apparatus which is closer to the user and the term "distal" refers to the end of the apparatus which is further from the user.

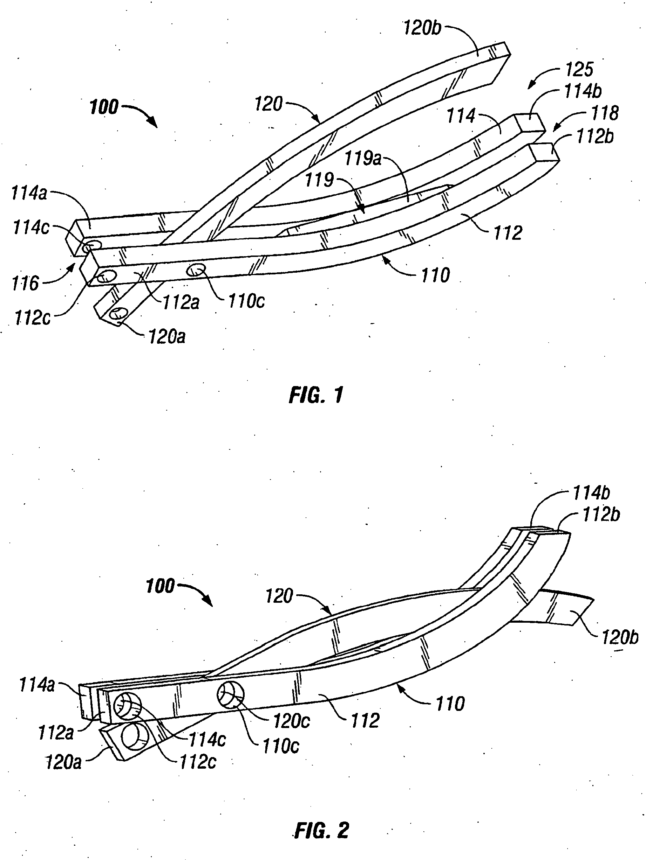

Referring to FIGS. 1-5F, the polyp removal jaws shown therein are generally designated as jaws 100. Jaws 100 comprise a first jaw member 110 and a second jaw member 120. First and second jaw members 110, 120 are pivotably connected to one another by a pivot pin 115. Jaw members 110, 120 are configured to retain, seal, cauterize, and/or sever tissue grasped therebetween. Jaw members 110, 120 are configured to engage tissue to be excised, such as stems of pedunculated polyps.

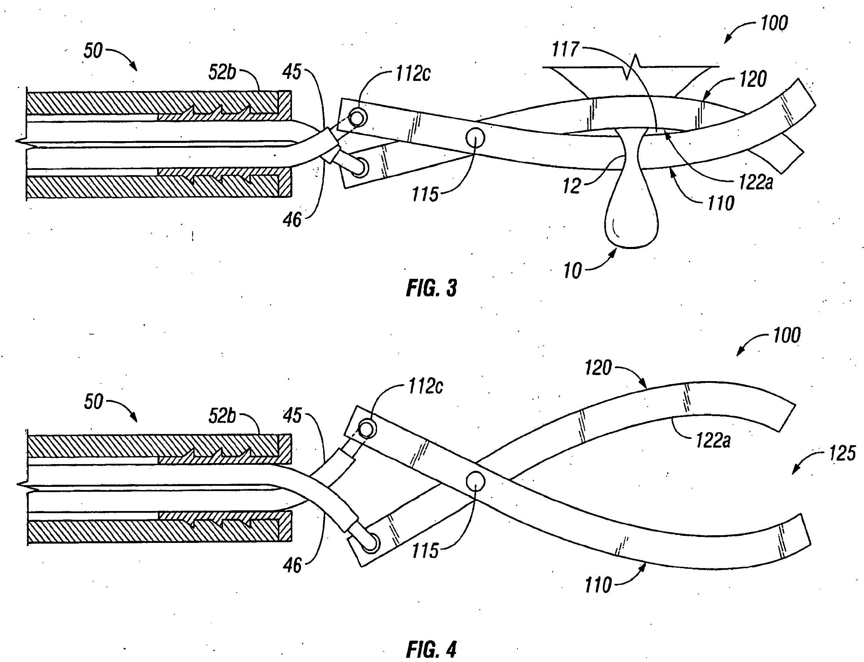

Jaws 100 may be incorporated into a hand-held instrument for use in open surgical procedures or may be configured, as shown in FIGS. 3 and 4, for incorporation into endoscopic instruments for use in closed surgical procedures. first and second jaw members 110, 120 may be composed of plastics, polymers, metals, alloys or the like or any combination thereof. First and second jaw members 110, 120 may be fabricated from any suitable dielectric material. Jaws 100 may be configured for monopolar and/or bipolar operation, as will be discussed below.

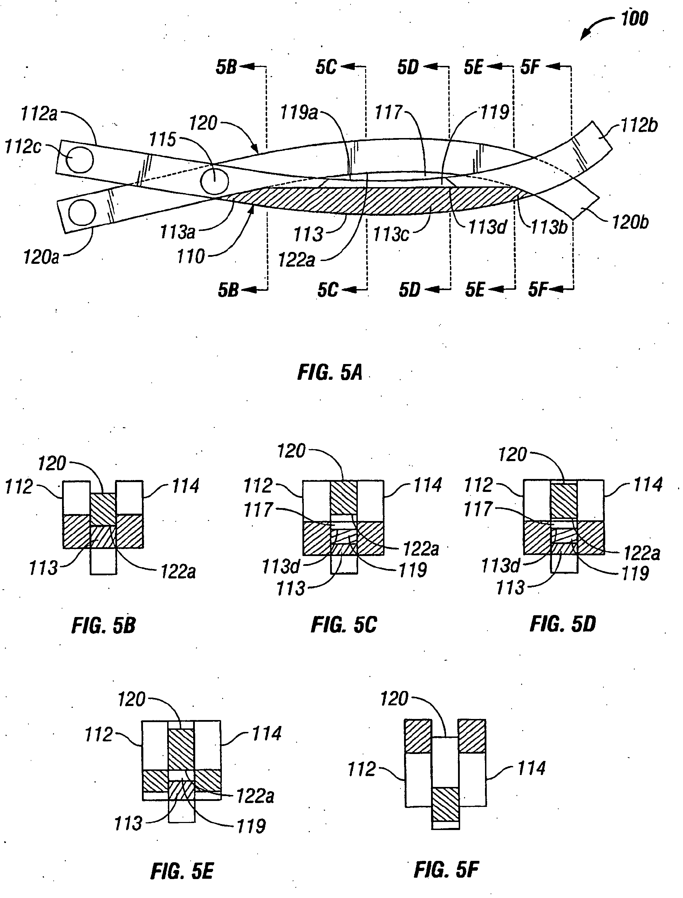

First jaw member 110 forms a semi-arcuate base including a first elongated section 112 and a second elongated section 114 spaced apart from substantially parallel to first elongate section 112. A connector or shelf member 113 (FIGS. 5A-5E) extends at least partially between first and second elongated sections 112, 114 to one another. First and second elongated sections 112, 114 include proximal ends 112a, 114a and distal ends 112b, 114b, respectively.

Proximal ends 112a, 114a of first and second elongated sections 112, 114 define a first channel 116 therebetween. First channel 116 is configured for selectively receiving a proximal end 120a of second jaw member 120 therein, as will be described in greater detail below. Distal ends 112b, 114b of elongated sections 112, 114, define a second channel 118 therebetween. Second channel 118 is configured for selectively receiving a distal end 120b of second jaw member 120 therethrough. Proximal ends 112a, 114a of first and second elongated sections 112, 114, respectively, define an opening 110c for receiving a pivot pin 115.

With continued reference to FIGS. 1-5F, second jaw member 120 forms a single semi-arcuate member having proximal and distal ends 120a, 120b, respectively. Proximal end 120a of second jaw member 120 is configured to be received within first channel 116 formed between proximal ends 112a, 114a of respective first and second elongated sections 112, 114, respectively. Proximal end 120a of second jaw member 120 defines opening 120c corresponding to openings 110c formed in first and second elongated section 112, 114. Opening 120c is configured for receiving pivot pin 115. Distal end 120 of second jaw member 120 is configured to be selectively received within and through second channel 118 formed between distal ends 112b, 114b of respective first and second elongated sections 112, 114.

Second jaw member 120 may comprise an electrically conductive material or may be fabricated substantially of an electrically conductive material. Second jaw member 120 may include one or more electrodes (not shown), supported thereon, for effecting monopolar and/or bipolar cutting and/or sealing of tissue. Second jaw member 120 may include an insulative layer (riot shown) to prevent a short circuit with first jaw member 110 and/or the one or more electrodes mounted thereon. The insulative layer may also prevent damage to tissue resulting from incidental contact during a surgical procedure.

Referring now to FIGS. 3-4 and 5A-5F, second jaw member 120 includes a tissue contacting surface 122a. Tissue contacting surface 122a may comprise at least a portion of a length of second jaw member 120. As will be discussed in more detail bellow, as first and second jaw members 110, 120 are closed about stem 12 of polyp 10 (FIG. 3), tissue contacting surface 122a of second jaw member 120 engages and guides stem 12 against first and second jaw members 110, 120. Tissue contacting surface 122a may have a rounded, sharpened, flattened or Other suitable transverse cross-sectional profile.

Proximal ends 112a, 114a of first and second elongated sections 112, 114, respectively, and proximal end 120a of second jaw member 120, may further be configured for incorporation into a conventional hand-held forceps or for operable engagement with the distal end of an endoscopic device. With particular reference to FIGS. 3 and 4, proximal ends 112a, 114a of first and second elongated sections 112, 114 define openings 112c, 114c (FIGS. 1 and 2) therein, configured for operable engagement with actuation cables 45, 46 extending from a distal end 52b of an endoscopic device 50. As will be described in more detail below, first and second actuation cables 45, 46 are configured to move first and second jaw members 110, 120. First and second actuation cables 45, 46 may operate in unison or independently of each other to pivot first and/or second jaw members 110, 120 relative to one another. Actuation cables 45, 46 may also be configured to supply electrosurgical energy to first and/or second jaw members 110, 120, respectively, and/or alternatively, to electrodes (not shown) mounted thereon.

Referring now, to FIGS. 5A-5F, shelf member 113 is disposed between first and second elongated section 112, 114. Shelf member 113 includes a proximal end 113a, a distal end 113b and a middle portion 113c therebetween. Proximal and distal ends 113a, 113b are configured to act as stops when first and second jaw members 110, 120 are in a closed and fully engaged position with one another. Proximal end 113a of shelf member 113 is configured to engage second jaw member 120 in a region proximal of tissue contacting surface 122a (FIGS. 5A and 5B). Distal end 113b of shelf member 113 is configured to engage second jaw member 120 in a region distal of tissue contacting surface 122a (FIGS. 5A and 5E). In this manner, a gap or opening 117 is formed between tissue contacting surface 122a of second jaw member 120 and an upper surface 113d of shelf member 113 of first jaw member 110 (FIGS. 5C and 5D). By varying the curvature of first and/or second jaw members 110, 120 and/or altering the configuration of proximal and/or distal ends 113a, 113b of shelf member 113, the height or dimensions of opening 117 may be adjusted. Proximal and distal ends 113a, 113b of shelf member 113 may include a layer of insulation to prevent a short circuit between first and second jaw members 110, 120.

Middle portion 113c of shelf member 113 may be recessed with, flush to or extend past an upper surface of first and second elongated sections 112, 114. Middle portion 113c may be flat, curved inwardly, curved outwardly, or may include a texture for more securely engaging tissue. Depending on the electrical configuration of jaw 100, middle portion 113c of shelf member 113 may include one or more electrodes 119 mounted thereon. Electrode 119 may be sized and dimensioned to be maintained on middle portion 113c of shelf member 113. Electrode 119 may be recessed within channels 116, 118 formed between first and second elongated sections 112, 114. Alternately, electrode 119 may be maintained flush with the top surface of elongated sections 112, 114 or may extend beyond the top surface of elongated sections 112, 114. Electrode 119 may have a flat, curved or textured tissue contacting surface 119a.

Referring back to FIG. 1 and 4, polyp removal jaw 100 is shown in a first or open position. In the open position, distal end 120b of second jaw member 120 is pivoted out from within channel 118 formed between distal ends 112b, 114b of first and second elongated sections 112, 114 to define an opening 125 between first and second jaw members 110, 120. Opening 125 is configured for facilitating the placement of jaws 100 about a portion of tissue, such as, for example the stem of a pedunculated polyp. Depending on the configuration of the actuation mechanism, and whether it is for open or closed procedures, first jaw member 110 may be held stationary relative to the actuation assembly (not shown) while second jaw member 120 is pivoted about pivot pin 115 relative to first jaw member 110. Both first and second jaw members may be pivoted relative to each, or second jaw member 120 may be held stationary relative to the actuation assembly while first jaw member 110 is pivoted about pivot pin 115 relative to second jaw member 120. First and second jaw member 110, 120 may be articulated up to and beyond ninety degrees (90°) relative to one another. The range of articulation of second jaw member 120 relative to first jaw member is limited only by range of motion of the actuation assembly connected thereto.

Turning now to FIGS. 2-3 and 5A-F, polyp removal jaw 100 is shown in a second or closed position. In the closed position, proximal and distal ends 110a, 110b of second jaw member 110 extend through or positioned in channels 116, 118, respectively, formed between elongated sections 112, 114. As described above, shelf member 113 includes proximal and distal ends 113a, 113b for engaging second jaw member 120. Gap 119 formed between first and second jaw members 110, 120 is configured for operably retaining stem 12 of a polyp 10 (FIG. 3). Whether of a monopolar or bipolar design, electrosurgical energy may be applied to either first or second jaw members 110, 120 or one or more electrodes 119 mounted thereon, at any time during the polyp removal procedure. For larger polyps it may be necessary to activate the tissue sealing mechanism prior to the complete closure of first and second jaw members 110, 120.

Referring now to FIGS. 6 and 7, polyp removal jaws according to an embodiment of the present disclosure are shown as 200. Polyp removal jaws 200 are substantially similar to polyp removal jaws 100 and will only be described to the extent necessary to disclose the difference in construction and operation between the two. Polyp removal jaws 200 include first and second jaw members 210, 220. Second jaw member 220 includes a distal end 220b configured for supplying electrosurgical energy by way of an electrode tip 222. When jaws 200 are in a closed position, as shown in FIG. 7, distal end 220b of second jaw member 220 extends through channel 218 of first jaw member 210 and defines an operational end 221 that acts as a monopolar pencil. Operational end 221 may be an extension of electrically conductive second jaw member 220. Operational end 221 includes an electrode or electrode tip 222. Electrode tip 222 may be selectively energized to permit a user to spot cauterize tissue without having to introduce a second instrument into the surgical field.

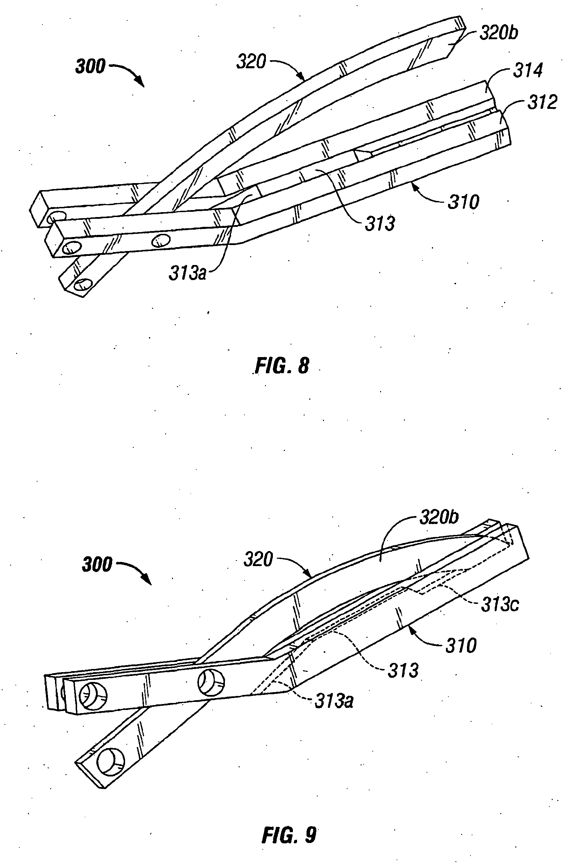

Referring now to FIGS. 8-10E, another polyp removal jaw 300 is illustrated. Polyp removal jaws 300 are substantially similar to polyp removal jaws 100, 200 and will only be described with respect to the difference in construction and operation therebetween. Polyp removal jaw 300 includes a first and second jaw member 310, 320. First jaw member 310 forms a level, or planar, or linear base including a first and second elongated section 312, 314 and a shelf 313 therebetween. In the present embodiment, shelf 313 extends distally between first and second elongated members 312, 314, substantially along an entire length of elongated members 312, 314, thereby preventing distal end 320b of second jaw member 320 from extending through elongated members 312, 314.

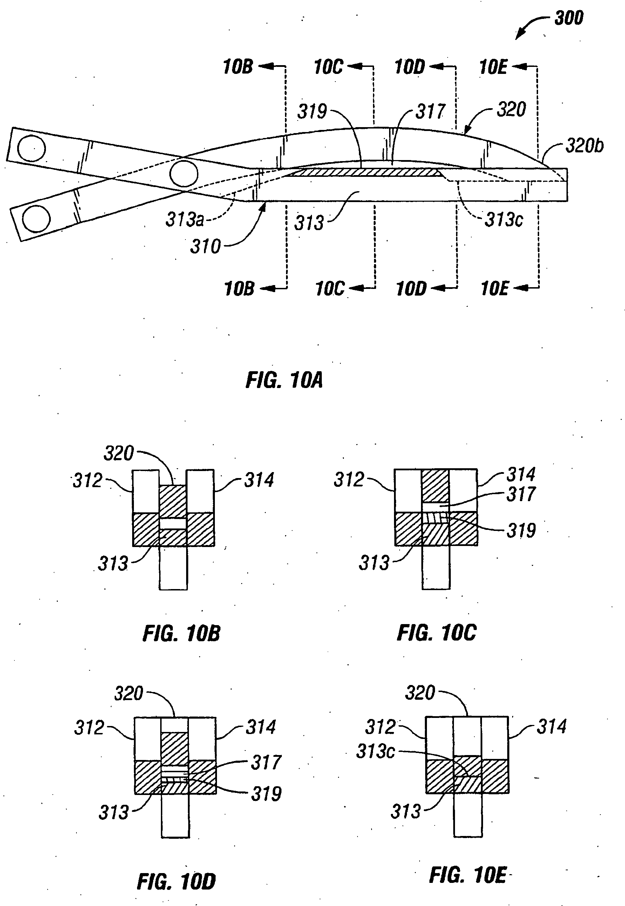

Turning now to FIGS. 10A-10E in particular, shelf member 313 is disposed between first and second elongated section 312, 314. Shelf member 313 extends distally the length of first and second elongated section 312, 314. Shelf member 313 defines a closure surface 313c configured to engage a distal end 320b of second jaw member 320 (FIGS. 10A and 10E) when jaws 300 are in a closed position. Proximal end 313a of shelf member 313 may be configured to engage a portion of second jaw member 320, or as shown (FIGS. 10A and 10B), proximal end 313a does not engage any portion of second jaw member 320. Shelf member 313 may include an electrode 319 mounted thereon (FIGS. 10A and 10C-D). Electrode 319 may be configured similar to electrode 119, as detailed above. A gap 317 is formed between first and second jaw members 310, 320. The height or dimension of gap 317 may vary depending on the configuration, i.e. arc, length, of first and second jaw members 310, 320.

Closure surface 313c of shelf 313 may be configured to securely engaged distal end 320b of second jaw member 320. Alternatively, closure surface 313c may be configured to complete a circuit upon contact with distal end 320b of second jaw member 320. Second jaw member 320 may be configured to deform as distal end 320b engages closure surface 313c of shelf 313: In this manner, the height of gap 317 between first and second jaw member 310, 320 will be reduced as first and second jaw members 310, 320 are squeezed together. Polyp removal jaw 300 may be monopolar, bipolar or a combination of the two.

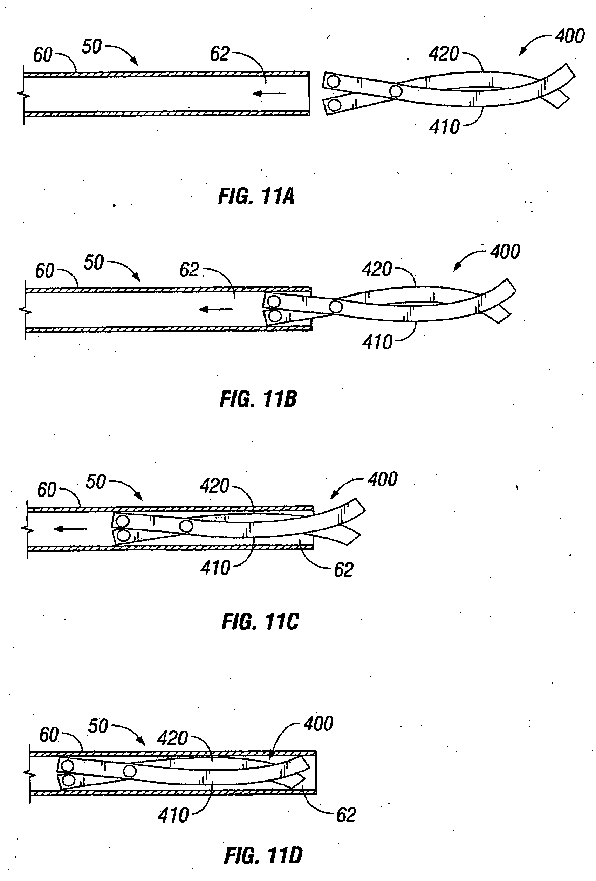

Referring now to FIGS. 11A-11D, polyp removal jaws 100, 200, 300 herein described may be configured to be received with a housing 60 of an endoscopic device 50, even when on opening 62 in housing 60 is less then the height of the jaws in a first or closed state. Polyp removal jaws 400 may be configured from elastomeric material, shape memory metals, plastics or the like. Referring initially to FIG. 11A, polyp removal jaws 400 include first and seconde jaw member 410, 420. When jaws 400 are exposed or deployed from housing 60, the height of polyp removal jaw 400 is greater than the dimension of opening 62 of housing 60 from which it is to be received. A polyp removal jaw 400 is retracted into housing 50, first and second jaw members 410, 420 are cammed against a front edge of housing 60 so as to be extended and compressed towards one another (10B-11C) until polyp removal jaws 400 are dimensioned to be completely received within housing 60. Polyp removal jaws 400 are configured to return to their initial shape upon ejection or deployment from housing 60. Polyp removal jaws 400 may be configured in any manner herein described.

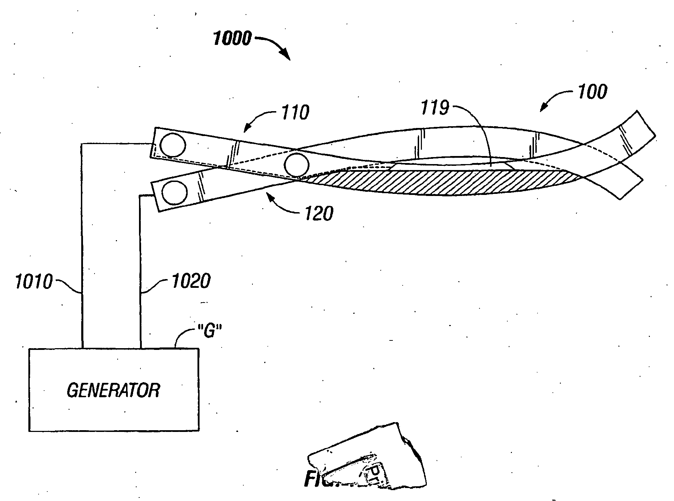

As seen in FIG. 12, any of the polyp removal jaws disclosed herein, such as, for example, polyp removal jaws 100 (as exemplarily shown in FIG. 12) may form a part of an electrosurgical system 1000. Electrosurgical system 100 may include at least an electrosurgical generator "G", and polyp removal jaws 100 electrically connected/connectable to electrosurgical generator "G" or the like via electrical conduits 1010, 1020. In particular, as seen in FIG. 12, a first electrical conduit 1010 is electrically connected to electrode 119 of first jaw 110 of polyp removal jaws 100, and a second electrical conduit 1020 is electrically connected to second jaw 120 of polyp removal jaws 100.

## Claims

1. An apparatus (200) for the removal of tissue comprising:
a first jaw member including first and second elongated members (210), the first and second elongated members having proximal and distal ends and defining a channel (218) therebetween; and
a second jaw member (220) having proximal and distal ends (220b) and defining a tissue contacting surface, wherein the distal end of the second jaw member is in a spaced apart relationship with the first jaw member when in a first position, and the distal end of the second jaw member extends into the channel defined between the distal ends of first and second elongated members when in a second position, and
the proximale end of the second jaw member being pivotably coupled between the proximal ends of the first and second elongated members; the apparatus being **characterized in that**:
the distal end (221) of the second jaw member is configured as a pencil and extends beyond the distal end of first and second elongated members of the first jaw member when the jaws are in the second position to provide an operational end (221) that includes a selectively energizable electrode tip (222) for delivering electrosurgical energy in a monopolar configuration to spot cauterize tissue.

2. The apparatus of claim 1, wherein the first and second elongated members include a shelf (113) extending at least partially therebetween.

3. The apparatus of claim 2, wherein an electrode is supported on the shelf.

4. The apparatus of claim 3, wherein the shelf is configured to extend distally between the first and second elongated members to form a stop for engaging a distal end of the second jaw member when the jaws are in the second position.

5. The apparatus of claim 2, 3 or 4, wherein the seconde jaw member is configured to operably engage the shelf when the first and second jaw members are in a closed position.

6. The apparatus of claim 1, wherein a gap is formed between the first and second jaw members when the first and second jaw members are in a closed position.

7. The apparatus of any preceding claim, further comprising a housing for receiving the first and second jaw members therein, wherein the first and second jaw members are flexed for receipt within the housing.

8. The apparatus of any preceding claim, wherein an electrode is operably disposed between the first and second elongated members.

9. The apparatus of any preceding claim, wherein at least one of the first and second jaw members is made of a resilient material and includes a semi-arcuate shape.

10. The apparatus of any preceding claim, herein proximal ends of the first and second jaw members are configured to be operably engaged with an endoscopic device.

11. The apparatus as claimed in any one of the preceding claims and including a source of electrosurgical energy operably connected to at least one of the jaw members to deliver electrosurgical energy to the tissue.

## Patentansprüche

1. Vorrichtung (200)zur Entfernung von Gewebe, umfassend:
ein erstes Klemmbackenelement, das erste und zweite langgestreckte Elemente (210) aufweist, wobei die ersten und zweiten langgestreckten Elemente proximale und distale Enden aufweisen und einen Kanal (218) zwischen sich definieren; und
ein zweites Klemmbackenelement (220), das proximale und distale Enden (220b) aufweist und eine Gewebekontaktfläche definiert, wobei das distale Ende des zweiten Klemmbackenelements in einer beabstandeten Beziehung mit dem ersten Klemmbackenelement ist, wenn es in einer ersten Position ist, und das distale Ende des zweiten Klemmbackenelements erstreckt sich in den Kanal, der zwischen den distalen Enden der ersten und zweiten langgestreckten Elemente definiert ist, wenn es in einer zweiten Position ist, und
das proximale Ende des zweiten Klemmbackenelements schwenkbar zwischen den proximalen Enden der ersten und zweiten langgestreckten Elemente gekoppelt ist; wobei die Vorrichtung **dadurch gekennzeichnet ist, dass**:
das distale Ende (221) des zweiten Klemmbackenelements als Stift konfiguriert ist und sich über das distale Ende der ersten und zweiten langgestreckten Elemente hinaus erstreckt, wenn die Klemmbacken in der zweiten Position sind, um ein operatives Ende (221) bereitzustellen, das eine selektiv energetisierbare Elektrodenspitze (222) zum Abgeben von elektrochirurgischer Energie in einer monopolaren Konfiguration zum punktförmigen Kauterisieren von Gewebe aufweist.

2. Vorrichtung nach Anspruch 1, wobei das erste und das zweite langgestreckte Element eine Ablage (113) aufweisen, die sich mindestens teilweise dazwischen erstreckt.

3. Vorrichtung nach Anspruch 2, wobei eine Elektrode auf der Ablage gelagert ist.

4. Vorrichtung nach Anspruch 3, wobei die Ablage konfiguriert ist, sich distal zwischen den ersten und zweiten langgestreckten Elementen zu erstrecken, um einen Anschlag zum Eingriff mit einem distalen Ende des zweiten Klemmbackenelements zu bilden, wenn die Klemmbacken in der zweiten Position sind.

5. Vorrichtung nach Anspruch 2, 3 oder 4, wobei das zweite Klemmbackenelement konfiguriert ist, funktionsmäßig mit der Ablage in Eingriff zu treten, wenn die ersten und zweiten Klemmbackenelemente in einer geschlossenen Position sind.

6. Vorrichtung nach Anspruch 1, wobei ein Spalt zwischen den ersten und zweiten Klemmbackenelementen ausgebildet wird, wenn die ersten und zweiten Klemmbackenelemente in einer geschlossenen Position sind.

7. Vorrichtung nach einem der vorhergehenden Ansprüche, die ferner ein Gehäuse für das Aufnehmen der ersten und zweiten Klemmbackenelemente darin aufweist, wobei die ersten und zweiten Klemmbackenelemente zur Aufnahme innerhalb des Gehäuses gebogen sind.

8. Vorrichtung nach einem der vorhergehenden Ansprüche, wobei eine Elektrode betriebsmäßig zwischen den ersten und zweiten langgestreckten Elementen angeordnet ist.

9. Vorrichtung nach einem der vorhergehenden Ansprüche, wobei mindestens eines von den ersten und zweiten Klemmbackenelementen aus einem elastischen Material hergestellt ist und eine halbbogenförmige Gestalt aufweist.

10. Vorrichtung nach einem der vorhergehenden Ansprüche, wobei die proximalen Enden der ersten und zweiten Klemmbackenelemente konfiguriert sind, um funktionsmäßig mit einer endoskopischen Vorrichtung in Eingriff gebracht zu werden.

11. Vorrichtung nach einem der vorhergehenden Ansprüche und umfassend eine Quelle für elektrochirurgische Energie, die funktionsmäßig mit mindestens einem der Klemmbackenelemente verbunden ist, um elektrochirurgische Energie an das Gewebe abzugeben.

## Revendications

1. Appareil (200) pour l'enlèvement de tissu comprenant :
un premier élément formant mâchoire incluant des premier et second éléments allongés (210), les premier et second éléments allongés ayant des extrémités proximales et distales et définissant un canal (218) entre eux ; et
un second élément formant mâchoire (220) ayant des extrémités proximale et distale (220b) et définissant une surface entrant en contact avec un tissu, dans lequel l'extrémité distale du second élément formant mâchoire est dans une relation espacée avec le premier élément formant mâchoire quand il est dans une première position, et l'extrémité distale du second élément formant mâchoire s'étend dans le canal défini entre les extrémités distales des premier et second éléments allongés quand il est dans une seconde position, et
l'extrémité proximale du second élément formant mâchoire étant couplée de manière pivotante entre les extrémités proximales des premier et second éléments allongés ; l'appareil étant **caractérisé en ce que** :
l'extrémité distale (221) du second élément formant mâchoire est configurée comme un crayon et s'étend au-delà de l'extrémité distale des premier et second éléments allongés du premier élément formant mâchoire quand les mâchoires sont dans la seconde position pour fournir une extrémité opérationnelle (221) qui inclut une pointe d'électrode (222) pouvant être excitée de manière sélective pour délivrer de l'énergie électrochirurgicale dans une configuration monopolaire pour cautériser ponctuellement un tissu.

2. Appareil selon la revendication 1, dans lequel les premier et second éléments allongés incluent un rebord (113) s'étendant au moins partiellement entre eux.

3. Appareil selon la revendication 2, dans lequel une électrode est supportée sur le rebord.

4. Appareil selon la revendication 3, dans lequel le rebord est configuré pour s'étendre de façon distale entre les premier et second éléments allongés pour former une butée pour mettre en prise une extrémité distale du second élément formant mâchoire quand les mâchoires sont dans la seconde position.

5. Appareil selon la revendication 2, 3 ou 4, dans lequel le second élément formant mâchoire est configuré pour mettre en prise de manière opérationnelle le rebord quand les premier et second éléments formant mâchoire sont dans une position fermée.

6. Appareil selon la revendication 1, dans lequel un espacement est formé entre les premier et second éléments formant mâchoires quand les premier et second éléments formant mâchoires sont dans une position fermée.

7. Appareil selon l'une quelconque des revendications précédentes, comprenant en outre un logement pour recevoir les premier et second éléments formant mâchoires en son sein, dans lequel les premier et second éléments formant mâchoires sont fléchis pour réception à l'intérieur du logement.

8. Appareil selon l'une quelconque des revendications précédentes, dans lequel une électrode est disposée de manière opérationnelle entre les premier et second éléments allongés.

9. Appareil selon l'une quelconque des revendications précédentes, dans lequel au moins un des premier et second éléments formant mâchoires est fait d'une matière élastique et inclut une forme semi-curviligne.

10. Appareil selon l'une quelconque des revendications précédentes, dans lequel les extrémités proximales des premier et second éléments formant mâchoires sont configurées pour être en prise de manière opérationnelle avec un dispositif endoscopique.

11. Appareil selon l'une quelconque des revendications précédentes, et incluant une source d'énergie électrochirurgicale connectée de manière opérationnelle à au moins un des éléments formant mâchoires pour délivrer de l'énergie électrochirurgicale au tissu.
